# EUROPEAN PATENT APPLICATION

(11) **EP 4 438 110 A1**
(43) Date of publication of application: **02.10.2024**
(21) Application number: 23190019.2
(22) Date of filing: 07.08.2023
(51) Int. Cl.: A61N 5/10, A61B 6/40

(54) **RADIATION THERAPY SYSTEM AND METHOD FOR MONITORING A SUBJECT DURING A RADIATION THERAPY**

(71) Applicant: Siemens Healthineers AG, 91301 Forchheim (DE)
(72) Inventor: Baer-Beck, Matthias, 91080 Spardorf (DE); Hofmann, Christian, 91058 Erlangen (DE)
(74) Representative: Siemens Healthineers Patent Attorneys

(57) **Abstract**

Radiation therapy system and method for monitoring a subject during a radiation therapy

A radiation therapy system comprising a treatment area (4) configured such that a subject (5) or part of a subject (5) can be placed within the treatment area, a static computed tomography system (1) comprising x-ray sources (15) and detector elements distributed around the treatment area (4), and a radiation therapy device (2) configured to create pulsed treatment beams (21) aiming at the treatment area (4) with a pulsed beam pattern comprising pulse duration times (22) of individual pulses and inter-pulse times (23) between pulses; wherein the computed tomography system (1) is configured to acquire scan data from the subject (5) or part of the subject (5) during the inter-pulse times (23).

## Description

The invention relates to a radiation therapy system, method for monitoring a subject or part of a subject during a radiation therapy, and a corresponding computer program.

Independent of the grammatical term usage, individuals with male, female or other gender identities are included within the term.

In radiation therapy, treatment is often planned based on computed tomography (CT). A CT scan for patient simulation typically represents the starting point for an organ and tumour segmentation as well as for dose calculation. In the state of the art, technical advances are paving the way towards improvements of the radiation therapy, such as an increase in accuracy and precision, shorter treatment times (e.g., hypo-fractionated and/or even flash therapy), and patient-individualized therapy concepts. In this context, it is desirable to provide a therapy workflow that is adaptive based on the current patient anatomy which may change during the course of the treatment. Such an adaptive therapy workflow is a prerequisite to enable high-precision radiation therapy with improved tumour control and reduced negative impact on the patient, e.g., reduced toxicity. In order to improve the precision of the radiation therapy, it is desirable to be able to verify, track, control and/or selectively steer the dose delivery during the treatment. For this purpose, the capability of online-imaging, i.e. imaging during or around the time of the treatment, the treated anatomy of the patient with a high temporal resolution, e.g. a temporal resolution of less than 0,5 seconds, and a high soft tissue contrast in the treatment isocentre could be very helpful or even essential.

CT imaging is generally capable to provide an imaging with sufficient image contrast. However, an online imaging of radiation therapy via CT imaging has thus far not been implemented to the knowledge of the inventors. There are several drawbacks that are related to an implementation of online-imaging via CT. Computed tomography systems are typically characterized by a fast-rotating x-ray source and detector in order to provide highly temporally resolved images with a distinct anatomical representation. On the other hand, the radiation device typically also rotates during treatment, but with a slower pace (e.g., on a time scale of more than 8 seconds). Using the CT system at the same time as the radiation therapy device typically leads to vibrations caused by the CT gantry that can negatively impact the precision of the radiation therapy device. Furthermore, the rotating CT x-ray source and detector may get in the way of the treatment device due to its rotation. Also, the CT measurement itself may be negatively impacted by the radiation caused by the radiation device. In particular, the x-rays from the radiation therapy may render at least part of the CT data useless such that no meaningful image can be reconstructed.

It is therefore an object of the invention to provide a means to improve the imaging for radiation therapy. For example, it would be desirable, to provide an imaging that can be performed during or temporarily close to a radiation therapy and that can provide a good imaging contrast of the treated area.

This object is met or exceeded by a system according to claim 1, a method according to claim 10, and a computer program according to claim 15. Further advantages and features result from the dependent claims, the description and the attached figures.

According to a first aspect of the invention, a radiation therapy system is provided. The radiation therapy system comprises a treatment area configured such that a subject or part of a subject can be placed within the treatment area, a static computed tomography system comprising x-ray sources and detector elements distributed around the treatment area, and a radiation therapy device configured to create pulsed treatment beams aiming at the treatment area with a pulsed beam pattern comprising pulse duration times of individual pulses and inter-pulse times between pulses. The computed tomography system is configured to acquire scan data from the subject or part of the subject during the inter-pulse times.

The radiation therapy device may be configured to be rotatable during a radiation therapy. For example, the radiation therapy device may be configured to rotate into another rotational position during inter-pulse times. The radiation therapy device may be configured to be non-rotating during the pulse duration time. Alternatively, the radiation therapy device may, for example, be configured to rotate continuously, both, during the pulse duration times and the inter-pulse times. The radiation therapy device may be a conventional radiation therapy device as known in the state of the art.

The static computed tomography system is in particular a computed tomography system where the x-ray sources and the detector elements are non-rotating during a computed tomography scan. Both, the x-ray sources and the detector elements are distributed around the treatment area. The computed tomography system is in particular configured to activate the x-ray sources that are distributed around the treatment area selectively to generate x-ray projections through the subject or part of the subject in the treatment area. Correspondingly, the x-rays may then be detected by the detector elements. For example, the x-ray sources may be distributed circularly around the treatment area. An axial direction may be defined to go orthogonally through an area that is spanned by the arrangement of the distributed x-ray sources. The detector elements may be distributed cylindrically around the treatment area. Hence, the distribution of the detector elements may extend in the axial direction. Preferably, in the axial direction, the detector elements are offset with respect to the x-ray sources. Using a static computed tomography system has the advantage that there does not have to be a rotation of the x-ray sources or the detectors during a CT scan. Hence, advantageously, rotation-caused vibrations that may negatively impact the radiation therapy can be avoided. Without the rotating component, the overall design of the system can be easier and/or more flexible. It can generally be easier to arrange both the radiation device and the CT system, when the CT system does not need the rotational component. In particular, since no fast rotations need to be enabled, the CT system can be built less massive and, therefore, in a more space-saving manner. The radiation therapy may thus, for example, be provided with a particularly compact and accessible design. Further advantageously, a high temporal resolution may be achievable, e.g. via fast switching between x-ray sources, without requiring a high mechanical stress due to a rotation and while having a lower technical challenge of data transmission (e.g., no slip ring is required) and power provision (no power supply via rotating parts. Also, without rotation, the amount of required power may be reduced.

The computed tomography system is configured to acquire scan data from the subject or part of the subject during the inter-pulse times. The scan data may, for example, be three-dimensional scan data. The subject may, for example, be a human being or an animal. The subject may, for example, be a patient. Part of the subject may be an anatomical part of the subject, such as a limb or an organ. Acquiring scan data during inter-pulse times means in particular that the scan data are acquired when there are currently no radiation therapy pulses. Advantageously, acquiring scan data during inter-pulse times may thus avoid an interference of x-rays from the radiation therapy with the CT measurement. This configuration is particularly possible due to the computed tomography system being a static computed tomography system. A rotating system cannot be controlled as well, since it would be at least very difficult and likely impossible to control the rotation on the time scale of typical radiation therapy pulses.

For example, the rotation can usually not be stopped on typical time scales of the radiation therapy pulses. For example, the pulse durations may typically be in the range of a few microseconds, such as 1 to 30 microseconds, preferably 10 to 20 microseconds. For example, the inter-pulse times may typically be in the general range of milliseconds, such as 0,1 to 20 milliseconds, preferably, 0,5 to 15 milliseconds, more preferably 1 to 10 milliseconds.

The computed tomography system may be configured to acquire data only during particular inter-pulse times. For example, the radiation therapy device may be configured to be applied in therapy units, each therapy unit comprising pulsed patterns with pulse durations and first inter-pulse times and longer second inter-pulse times being provided between the therapy units. The computed tomography system may, for example, be configured to acquire scan data during the second inter-pulse times. Advantageously, there may thus be more continuous time to acquire data. Hence, the image quality during this time may potentially be better. Additionally and/or alternatively, the computed tomography system may be configured to acquire scan data during the first inter-pulse times. Scan data from the first inter-pulse times may allow a monitoring even during therapy. Hence, for example, the therapy may be adjusted even during single therapy units, if necessary. For example, the system may be configured to acquire high-quality images during the second inter-pulse times and lower-quality images during the first inter-pulse times. This combination may allow a good image quality from the pause times between therapy units combined with the possibility to even monitor therapy during therapy units. Advantageously, the inventive system may enable to adapt new clinical workflows and/or allow for highly conformal treatments. For example, radiosurgery applications may be conceivable with such a system.

The radiation therapy system may comprise a control device that is configured to control the radiation therapy device and/or the computed tomography system. The control device may, for example, be a computer device, such as a medical control station, a personal computer, an online server, a tablet, or smartphone.

According to an embodiment, the computed tomography system is configured to reconstruct image data, in particular 3D image data, of the subject or part of the subject based on the scan data acquired during inter-pulse times. Hence, the system may be configured to use the scan data to reconstruct image data. The computed tomography system may be configured to provide real time imaging, preferably 3D real time imaging, during a radiation therapy. Real time imaging may, for example, be provided by reconstructing and updating the acquired scan data essentially immediately. Additionally or alternatively, the computed tomography system may be configured to transmit the acquired scan data to another device. The other device may, for example, be a device configured for image reconstruction.

According to an embodiment, the computed therapy system is configured to activate the x-ray sources only during inter-pulse times and pause acquisition during pulse duration times. Hence, the computed therapy system may be configured to switch of the x-ray sources or not to activate the x-ray sources during the pulse duration times. In other words, the computed tomography system may be configured to stop the CT scan data acquisition whenever the radiation therapy device switches on a treatment pulse. Once the treatment beam of the radiation therapy device is switched off again, i.e. during inter-pulse times, the computed tomography system can continue with the data acquisition with the next CT projection. Advantageously, specifically activating the x-ray sources during inter-pulse times is easier with a static computed tomography system than it would be with a computed tomography system of the third generation, i.e. a computed tomography system with rotating x-ray source and detector. This is due to the fact, that a static computed tomography system does not have to physically rotate heavy components for the CT scan.

In particular, it may advantageously be possible, that, except for the starting and stopping of the data acquisition, no further time synchronization between the CT data acquisition of the computed tomography system and the treatment beam of the radiation therapy device is needed. With a rotation-based system it would typically be necessary, to consider and optimize the dependencies between rotation time, detector integration time of the CT system and pulse duration time and inter-pulse time of the radiation therapy device. Generally, with a rotation-based system, it would typically be necessary, to optimize the time synchronization of such a system for each scan mode of the CT system and also for each treatment mode of the radiation therapy device. Using the static CT system, the timing control can be less complex since it may be reduced to a simple on/off switch of the x-ray sources of the CT system and the acquisition of the scan data can be simply re-started right from the position (projection) where it was stopped without generating any sampling gaps or without any complex scan procedures which would may otherwise be needed to avoid those gaps. Further advantageously, not activating the x-ray sources during pulse-duration times may reduce the imaging radiation dose on the subject. For example, assuming 1 millisecond of inter-pulse times and 250 ps of pulse duration times, the imaging radiation dose may be reduced by about 20%.

According to an embodiment, the radiation therapy system is configured to discard scan data acquired during pulse duration times. This may be an alternative to not acquiring scan data during pulse duration times. While the advantageous effect of reducing the radiation dose may not be achieved with this alternative, this variant can still have the advantage of ensuring that the acquired CT scan data that are not discarded are not disturbed by the treatment beam.

According to an embodiment, the radiation therapy system is configured to interleave individual treatment pulses with groups of computed tomography projections such that each group comprises at least one computed tomography projection, preferably a plurality of computed tomography projections, wherein a computed tomography projection comprises an activation of at least one of the x-ray sources to create at least one x-ray beam and a detection of the least one x-ray beam projected through the treatment area by at least one of the detector elements. The beam is in particular projected through the subject or part of the subject. The created at least one x-ray beam may, for example, be a fan beam. The fan may be configured such that the subject and/or the part of the subject and/or the treated area is within the fan beam. Preferably, the groups of computed tomography projections may be configured such that the duration of all the projections of one group essentially corresponds to the duration of the inter-pulse time. Preferably, the groups of computed tomography projections may be configured such the first projection of one group starts essentially at the beginning of the inter-pulse time. Depending on the duration of the inter-pulse time and the requirements of the CT scan, e.g. the needed or optimal duration of one projection, there may be one or more than one projections in one group. Interleaving the treatment pulses with the groups may thus mean that the acquisition with the static computed tomography system is paused during pulse duration times, i.e. when the treatment beam is switched on and, once the treatment beam is switched off, the data acquisition with the static computed tomography system continues with the next projection. The time intervals between projections within one group may be constant across the group. Advantageously this embodiment may enable an efficient use of available time during the inter-pulse times, while also ensuring that no (mostly useless) data are acquired during the pulse duration times. Optionally, the computed tomography system may be configured such that projections are acquired one after another in virtual rotation direction of the static computed tomography system. Hence the acquisition may be corresponding to an acquisition with a rotating CT but with the omission of acquisition during pulse duration times. One full CT scan may comprise projections from multiple groups. It is conceivable, that a new full scan starts within one group. Hence, some projections of one group may belong to another full scan than other projections of the group or some projections may be used for the reconstruction of another image than other projections of the group, respectively.

According to an embodiment, the x-ray sources comprise field emission nanotube-based cathodes. Advantageously, field emission nanotube-based cathodes may provide a very good flexibility to be quickly activatable and de-activatable. For example, there is no large after-burn effect, as may be the case with heat-based cathodes. Furthermore, the technology of field emission nanotube-based cathodes has advantageously progressed in recent years so that building a static computed tomography system with distributed x-ray sources, e.g. in the form of an x-ray source array, can now be achieved with a power of about 10-15 kW. It can be expected that in the coming years this technology may even reach higher power values, potentially corresponding to the upper power values of conventional rotating computed tomography systems of the third generation.

According to an embodiment, the radiation therapy device comprises a medical linear accelerator. The radiation therapy device may be configured to produce high energy photons, in particular in the x-ray range, and/or electrons to produce the treatment beam. Preferably, the radiation therapy device may comprise an electron and photon linear accelerator, in particular a Megavolt (MV) electron and photon linear accelerator. Such a device may also be referred to as linac. For example, the radiation therapy device may accelerate electrons to collide with a heavy metal material in order to produce high-energy x-ray photons. The radiation therapy device may be configured to produce x-ray beams with a shape that correspond to a target, such as a patient's tumour.

According to an embodiment, the computed tomography system is configured to acquire scan data by consecutively activating the x-ray sources in the order that the x-ray sources are arranged around the treatment area. Hence, the activation of the x-ray sources may be configured to allow a scan data acquisition that corresponds to an acquisition of rotating CT systems. Hence, conventional methods for image reconstruction may be particularly easy to adapt for this embodiment. The x-ray sources may be spaced equidistantly around the treatment area. CT projections for the data acquisition may be placed azimuthally equidistantly and in fixed time intervals.

According to an embodiment, the computed tomography system is configured to acquire scan data by activating the x-ray sources in such an order that each two consecutively activated x-ray sources are spaced apart by more than 90° on a circle around the treatment area. Advantageously, the x-ray sources being spaced apart such may allow for a better correction of movement, since different angles can be monitored within small time frames.

According to a further aspect of the invention, a method for monitoring a subject or part of a subject during a radiation therapy, the radiation therapy involving pulsed treatment beams aiming at a treatment area with a pulsed beam pattern comprising pulse duration times of individual pulses and inter-pulse times between pulses, is provided. The method comprises the following steps:
(a) acquiring scan data with a computed tomography system, the computed tomography system comprising x-ray sources and detector elements distributed around the treatment area, from the subject or part of the subject during the inter-pulse times;
(b) reconstructing image data, in particular 3D image data, of the subject or part of the subject based on the scan data acquired during inter-pulse times.

All features and advantages of the system may be adapted to the method and vice versa. The computed tomography system that is applied may be a computed tomography system as described herein. The radiation therapy may be a radiation therapy with pulsed treatment beams common in the state of the art. Advantageously, the method may enable the monitoring of the subject or part of the subject even during the radiation therapy. Hence, a closer surveillance may be enabled with this method.

According to an embodiment, the method further comprises:
- in step (a), performing a first computed tomography scan by acquiring scan data from the subject or part of the subject during the inter-pulse times;
- in step (b), reconstructing image data based on the scan data from the first scan.

According to this embodiment, the method comprises the following additional steps:
- (c) after the first computed tomography scan, performing at least one second computed tomography scan, where the at least one second computed tomography scan comprises a lower number of computed tomography projections than the first computed tomography scan; and
- (d) updating the image data based on the scan data from the at least one second computed tomography scan. The image data that are updated may in particular be the image data reconstructed in step b. Steps (c) and (d) may be applied multiple times. Accordingly, the image data that are updated may be the image data that have already been updated in a previous step. The at least one second computed tomography scan may be a low-resolution scan, such that the resolution of the second computed tomography scan is lower than the resolution of the first computed tomography scan. Advantageously, the second scan may allow to quickly update the data from the first scan, therefore allowing a higher temporal resolution of the updates while still having the high-resolution information of the first scan. Hence, low-resolution updates of the at least one second scan may be completed and/or combined with higher resolution data from the first scan. Accordingly, since a very high level of temporal resolution may be achievable, fast-moving objects may be imaged and/or monitored. Furthermore, artifacts due to patient movement may be better avoidable. Additionally and/or alternatively, the at least one second scan may advantageously allow for low-dose image updates. Hence, the radiation dose of the subject may be reduced. Advantageously, the scan data acquisition of the at least one second scan may be combined with a compressed sensing approach. Compressed sensing is generally known in the state of the art. It is based on acquiring a reduced amount of data such that data with no or little information is left out in order to get compressed approximated data.

The scan data from the at least one second computed tomography scan, in particular from a plurality of second computed tomography scans, may be used to track an object, such as a tumour. Movement of the object may be calculated from the image data acquired with the first and second scans. For example, markers may be used for tracking the object, wherein the markers are estimated from the projections of the at least one second computed tomography scan. For the calculating the tracking a method existing in the state of the art may be applied, such as the method described by Ross I Berbeco et al. in "Integrated radiotherapy imaging system (IRIS): design considerations of tumour tracking with linac gantry-mounted diagnostic x-ray systems with flat-panel detectors", Phys. Med. Biol. 49 (2004) 243-255. Further methods that may be applied are described by Toshiyuki Harada et al. in "Real-Time Tumor-Tracking Radiation Therapy for Lung Carcinoma by the Aid of Insertion of a Gold Marker Using Bronchofiberscopy", Cancer. 2002 Oct 15;95(8):1720-7. doi: 10.1002/cncr.10856. PMID: 12365020 and by Ye Zhang et al. in "Online image guided tumour tracking with scanned proton beams: a comprehensive simulation study", Phys. Med. Biol. 59 (2014) 7793-7817.

According to an embodiment, the computed tomography projections of the at least one second computed tomography scan are directed at a region of interest that is a sub-region of the region covered by the computed tomography projections of the first computed tomography scan. Hence a sampling density may be optimized to the region of interest. Advantageously, local updates can thus be achieved with a high temporal resolution.

According to an embodiment, the lower number of computed tomography projections from the at least one second computed tomography scan is performed by activating a sub-group of the x-ray sources of the computed tomography system; wherein the sub-group of the x-ray sources is chosen such that the distances between nearest neighbours of x-ray sources is equidistant across the sub-group. Advantageously an even distribution of scan data may thus be acquired.

According to an alternative embodiment, the method further comprises
- in step (a), performing an initial computed tomography scan by acquiring scan data from the subject or part of the subject during the inter-pulse times with a low number of computed tomography projections;
- in step (b), reconstructing rough image data based on the scan data from the first scan in order to determine an estimate of a position of a region of interest;
- (c) after the initial computed tomography scan, performing at least one further computed tomography scan, wherein the at least one further computed tomography scan comprises further computed tomography projections that are optimized to image the region of interest;
- (d) reconstructing image data based on the scan data from the at least one further computed tomography scan.

Hence, advantageously, a rough estimate of the position of the region of interest may be determined. The region of interest may, for example be the region where a tumour is located. The low number of computed tomography projections of the initial scan may for example be 2 to 4 projections, preferably 2 to 3 projections, more preferably 2 projections. The two projections may be orthogonal with respect to each other. While the low number of projections may not be enough to get diagnostic information, the low number of projections may still allow to estimate the position of the region of interest. The further computed tomography projections may be adapted to efficiently image the region of interest. Adapting the further computed tomography projections may comprise adjusting the angle of the further computed tomography projections. For example, the angle of the further computed tomography projections may be adjusted such that the projections comprise projections that correspond to the main symmetry axes of an object to be examined in the region of interest. The object may, for example, be a tumour. Optionally, in addition to the projections that correspond to the main symmetry axes of the object, at least one additional projection may be provided. The at least one additional projection may be chosen such that the resolution is increased along a direction of interest. In some cases, it may be advantageous, to have an increased resolution along a specific direction. The specific direction may in particular be related to the radiation therapy.

According to a further aspect of the invention, a computer program is provided. The computer program comprises instructions which, when the program is executed by a control unit of a computed tomography system, cause the computed tomography system to carry out the method according to one of the embodiments as described herein. All features and advantages of the system and of the method may be adapted to the computer program and vice versa.

According to a further aspect of the invention, a computer-readable storage medium, in particular non-transient storage medium, is provided. The computer-readable storage medium comprises instructions which, when executed by a control unit of a computed tomography system, cause the computed tomography system to carry out the method according to one of the embodiments as described herein. All features and advantages of the system, of the method, and of the computer program may be adapted to the computer-readable storage medium and vice versa.

The embodiments described herein may be combined with each other unless indicated otherwise.

The accompanying drawings illustrate various exemplary embodiments and methods of various aspects of the invention.
Fig. 1 shows a radiation therapy system according to an embodiment of the invention.;
Fig. 2 shows a flow diagram of a method for monitoring a subject or part of a subject during a radiation therapy according to an embodiment of the invention;
Fig. 3 shows an interleave pattern of treatment beams and computed tomography projections according to an embodiment of the invention;
Fig. 4 shows a pattern of treatment beams and computed tomography projections that are not interleaved but where the computed tomography projections are applied continuously;
Fig. 5 shows a cross section of a computed tomography system with a pulsing scheme according to an embodiment of the invention;
Fig. 6 shows a cross section of a computed tomography system with a pulsing scheme according to a further embodiment of the invention;
Fig. 7 shows a cross section of a computed tomography system with a pulsing scheme according to a further embodiment of the invention;
Fig. 8 shows a flow diagram of a method for monitoring a subject or part of a subject during a radiation therapy according to a further embodiment of the invention;
Fig. 9 shows a flow diagram of a method for monitoring a subject or part of a subject during a radiation therapy according to a further embodiment of the invention;
Fig. 10 shows a computed tomography pulsing scheme according to an embodiment of the invention; and
Fig. 11 shows a computed tomography pulsing scheme according to an embodiment of the invention.

Similar elements are designated with the same reference signs in the drawings.

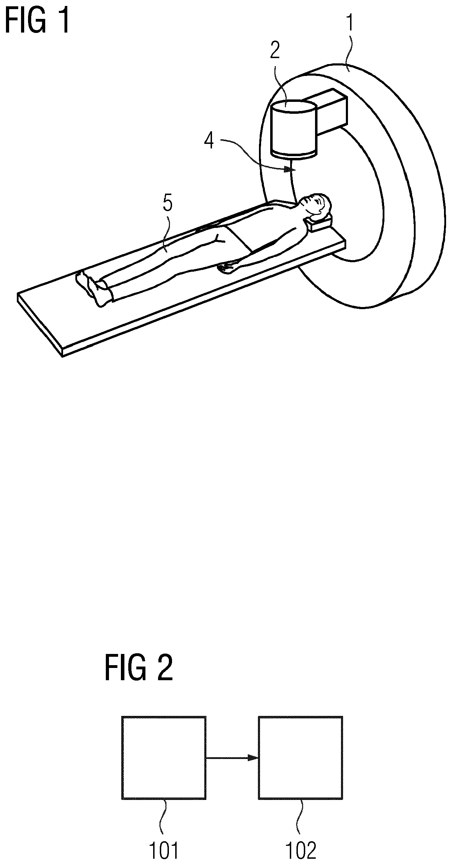

Figure 1 shows a radiation therapy system according to an embodiment of the invention. The radiation therapy system comprises a treatment area 4 that is configured such that a subject 5 or part of a subject 5 can be placed within the treatment area 4. The radiation therapy system further comprises a static computed tomography system 1 comprising x-ray sources and detector elements distributed around the treatment area 4. Preferably, the x-ray sources may comprise field emission nanotube-based cathodes. The radiation therapy system further comprises a radiation therapy device 2 configured to create pulsed treatment beams 21 aiming at the treatment area 4 with a pulsed beam pattern comprising pulse duration times of individual pulses and inter-pulse times 23 between pulses. The radiation therapy device 2 comprises a medical linear accelerator. Preferably, the radiation therapy device 2 may comprise an electron and photon linear accelerator, in particular a Megavolt electron and photon linear accelerator. The computed tomography system 1 is configured to acquire scan data from the subject 5 or part of the subject 5 during the inter-pulse times 23. A corresponding method that the computed tomography system 1 is configured to apply is shown in figure 2.

Figure 2 shows a flow diagram of a method for monitoring a subject 5 or part of a subject 5 during a radiation therapy according to an embodiment of the invention. The radiation therapy involves pulsed treatment beams 21 aiming at a treatment area 4 with a pulsed beam pattern comprising pulse duration times of individual pulses and inter-pulse times 23 between pulses. The method itself comprises a first step 101 of acquiring scan data with a computed tomography system 1, the computed tomography system 1 comprising x-ray sources and detector elements distributed around the treatment area 4, from the subject 5 or part of the subject 5 during the inter-pulse times 23. In a further step 102, image data of the subject 5 or part of the subject 5 are reconstructed based on the scan data acquired during inter-pulse times 23. The image data may, in particular, be 3D image data.

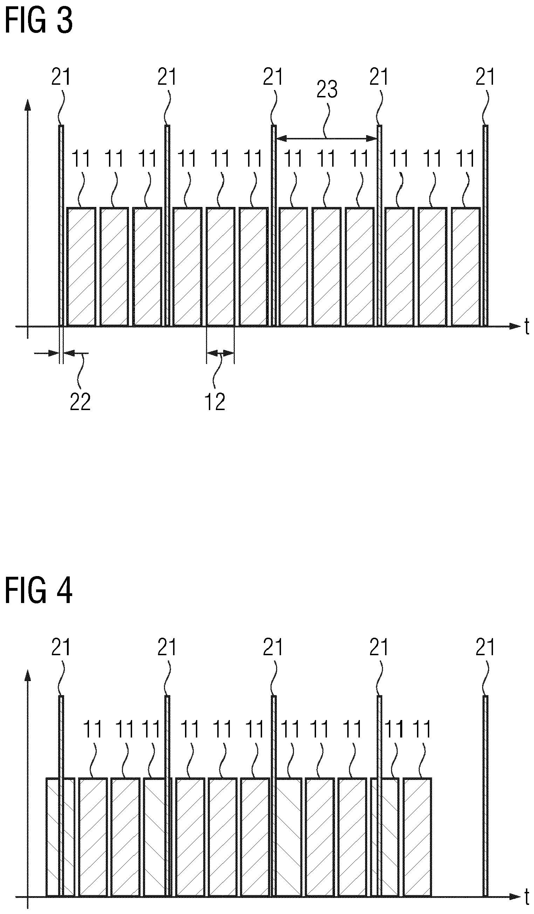

Preferably, the radiation therapy system 1 may be configured to interleave individual treatment pulses with groups of computed tomography projections 11 such that each group comprises at least one computed tomography projection 11, preferably a plurality of computed tomography projections 11. A computed tomography projection 11 comprises an activation of at least one of the x-ray sources to create at least one x-ray beam and a detection of the least one x-ray beam projected through the treatment area 4 by at least one of the detector elements. Figure 3 shows a corresponding interleave pattern of treatment beams 21 and computed tomography projections 11. In this representation, the horizontal axis is a time axis. As can be seen, in this embodiment, the computed therapy system 1 is configured to activate the x-ray sources only during inter-pulse times 23 and pause acquisition during pulse duration times 22. In this embodiment, the inter-pulse times 23 are in the order of magnitude of about 1 millisecond and the pulse duration times are in the order of magnitude of about 15 microseconds. Furthermore, in this embodiment, the projection duration 12 of the computed tomography projections 11 is in the order of magnitude of about 250 microseconds. Here, there are three computed tomography projections 11 within each inter-pulse time 23. Due to the fact that the components of the static computed tomography system 1 do not rotate physically it is quite straight forwardly achievable to interleave the projections 11 and the treatment beams 21 in order to avoid scattered radiation from the treatment beams 21 on the detector(s) of the static computed tomography system 1. For example, the projections 11 for imaging can be placed radially equidistantly and in fixed temporal intervals. With a rotating computed tomography system this is usually not possible in the same straightforward way. The computed tomography system 1 is configured to reconstruct image data of the subject 5 or part of the subject 5 based on the scan data acquired during the inter-pulse times 23.

Figure 4 shows a pattern of treatment beams 21 and computed tomography projections 11 that are not interleaved but where the computed tomography projections 11 are applied continuously. Projections 11 that overlap with a treatment beam 21 can usually not be used for reconstruction because they would be corrupted due to scatter from the treatment beam 21. In this example, about 4 out of 12 projections 11 are thus affected by treatment beams 21. A rotating computed tomography system might produce such a pattern. With a rotating computed tomography system, a synchronization of the computed tomography data is generally not achievable as straightforwardly as shown in the example of figure 3. With a fixed projection integration timing of a rotating computed tomography system, a continuous real time acquisition would therefore corrupt a portion of the projections 11 because they would be corrupted through scatter from the treatment beams 21. In this example, with inter-pulse times 23 of about 1 millisecond, roughly every 4th projection 11 will need to be "left" out because otherwise projections 11 would have scattered radiation of the MV treatment beams 21. Thus, for a rotating computed tomography system, sample gaps occur that do not have to occur when using a static computed tomography system 1. Since the image quality of CT images strongly depends on the number of acquired projections 11 per half rotation, sampling gaps in the projection direction reduce the achievable image quality. This effect typically increases for faster rotation times, assuming a fixed integration time, in this example of 250 microseconds. For faster rotation times the number of acquired projections 11 per half rotation is lower and therefore sampling gaps would have relatively more impact on the final image quality. The same holds true also for the case where the pulsing rate of the treatment beam 21 is increased. Even then the number of sampling gaps is increased on a rotation-based computed tomography system. This disadvantage may be avoided by using a static computed tomography system 1 as imaging device on a combined radiation therapy system.

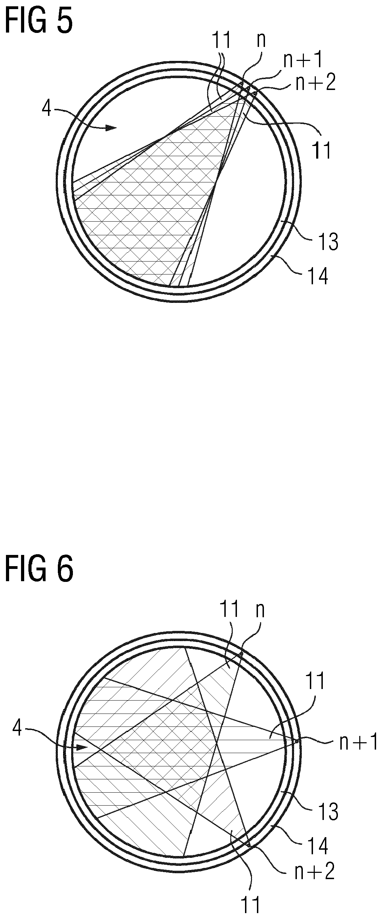

Figures 5-7 each show a cross section of a computed tomography system 1 according to embodiments of the invention, each cross section comprising a detector arrangement 13 and of an array 14 of the x-ray sources 15 of the computed tomography system 1. Figure 5 shows a pulsing scheme of a computed tomography system 1 according to an embodiment of the invention. Here, the computed tomography system 1 is configured to acquire scan data by consecutively activating the x-ray sources 15 in the order that the x-ray sources 15 are arranged around the treatment area 4. The data acquisition of this pulsing scheme is structurally similar to a data acquisition by a rotating computed tomography system. This pulsing scheme may be referred to as standard rotation with dense sampling. Figure 6 shows a pulsing scheme of a computed tomography system 1 according to another embodiment of the invention. In this embodiment, a lower number of computed tomography projections 11 is performed by activating a sub-group of the x-ray sources 15 of the computed tomography system 1. Here, the sub-group of the x-ray sources 15 is chosen such that the distances between nearest neighbours of x-ray sources 15 is equidistant across the sub-group. Compared to the pulsing scheme shown in figure 5, some of the x-ray sources 15 are not activated here. This pulsing scheme may be referred to as sparse equidistant sampling. Figure 6 shows a pulsing scheme of a computed tomography system 1 according to another embodiment of the invention. In this embodiment, the computed tomography system 1 is configured to acquire scan data by activating the x-ray sources 15 in such an order that each two consecutively activated x-ray sources 15 are spaced apart by more than 90° on a circle around the treatment area 4.

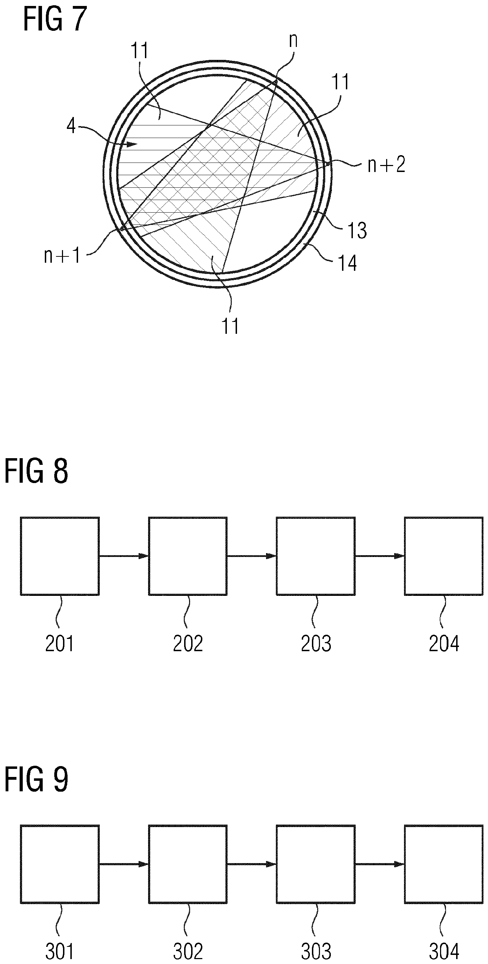

Figure 8 shows a flow diagram of a method for monitoring a subject 5 or part of a subject 5 during a radiation therapy according to a further embodiment of the invention. The radiation therapy involves pulsed treatment beams 21 aiming at a treatment area 4 with a pulsed beam pattern comprising pulse duration times of individual pulses and inter-pulse times 23 between pulses. The method itself is performed using a computed tomography system 1 that comprises x-ray sources 15 and detector elements distributed around the treatment area 4. The method comprises a first step 201 of performing a first computed tomography scan with the computed tomography system 1 and acquiring scan data from the subject 5 or part of the subject 5 during the inter-pulse times 23. In a further step 202, image data of the subject 5 or part of the subject 5 are reconstructed based on the scan data acquired during the inter-pulse times 23 of the first scan. The image data may, in particular, be 3D image data. In a further step 203, performed after the step 201 including the first computed tomography scan, at least one second computed tomography scan is performed. The at least one second computed tomography scan comprises a lower number of computed tomography projections 11 than the first computed tomography scan. For example, a lower number of computed tomography projections 11 may be performed as shown in figure 6. Hence, a sub-group of the x-ray sources 15 of the computed tomography system 1 may be activated. Optionally, the sub-group of the x-ray sources 15 may be chosen such that the distances between nearest neighbours of x-ray sources 15 is equidistant across the sub-group. However, other arrangements of the CT pulsing scheme. are possible as well. For example, the computed tomography projections 11 of the at least one second computed tomography scan may be directed at a region of interest 41 that is a sub-region of the region covered by the computed tomography projections 11 of the first computed tomography scan. Hence, a region of interest 41, i.e. the sub-region, may be scanned with the second scan, while the first scan is directed at a region with a greater area. In a further step 204, the image data are updated based on the scan data from the at least one second computed tomography scan. Hence, advantageously, the second scan may be faster than the first scan due to the smaller number of projections 11 and may thus allow to achieve a greater temporal resolution. On the other hand, the first scan may allow to also have more detailed information and/or information from a grater area.

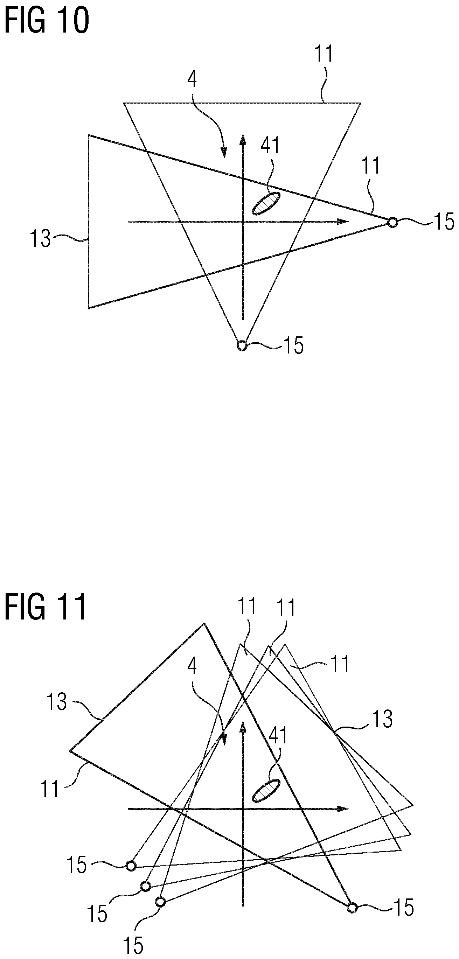

Figure 9 shows a flow diagram of a method for monitoring a subject 5 or part of a subject 5 during a radiation therapy according to a further embodiment of the invention. The radiation therapy involves pulsed treatment beams 21 aiming at a treatment area 4 with a pulsed beam pattern comprising pulse duration times of individual pulses and inter-pulse times 23 between pulses. The method itself is performed by using a computed tomography system 1 comprising x-ray sources 15 and detector elements distributed around the treatment area 4. The method comprises a first step 301 of performing an initial computed tomography scan by acquiring scan data with the computed tomography system 1 from the subject 5 or part of the subject 5 during the inter-pulse times 23 with a low number of computed tomography projections 11. For example, the low number of projections 11 my consist of two projections 11. One example of this is shown in figure 10, where two computed tomography projections 11, originating from two different x-ray sources 15, are directed at the treatment area 4 that comprises a region of interest 41 that is part of a subject 5. The projections 11 are detected by the detector arrangement 13 of the of the computed tomography system 1, comprising the detector elements. Hence, the region of interest 41 may be defined by an object, e.g. a tumour of the subject 5. In a further step 302, image data of the subject 5 or part of the subject 5 are reconstructed based on the scan data acquired during inter-pulse times 23 in order to (roughly) determine an estimate of a position of the region of interest 41. The image data may, in particular, be 3D image data. In a further step 303, performed after the step 301 including the initial computed tomography scan, at least one further computed tomography scan is performed. The at least one further computed tomography scan comprises further computed tomography projections 11 that are optimized to image the region of interest 41. An example of a corresponding CT pulsing scheme is shown in figure 11. Based on the estimated position of the region of interest 41 the further computed tomography projections 11, originating from different x-ray sources 15, are set to image the region of interest 41. Preferably, the further computed tomography projections 11 may correspond to the main symmetry axes of the object defining the region of interest 41. As shown in the example of figure 11, in addition to the computed tomography projections 11 corresponding to the main symmetry axes of the object, additional projections 11 may be used. In a further step 304, image data are reconstructed based on the scan data from the at least one further computed tomography scan.

## Claims

1. A radiation therapy system comprising
a treatment area (4) configured such that a subject (5) or part of a subject (5) can be placed within the treatment area,
a static computed tomography system (1) comprising x-ray sources (15) and detector elements distributed around the treatment area (4), and
a radiation therapy device (2) configured to create pulsed treatment beams (21) aiming at the treatment area (4) with a pulsed beam pattern comprising pulse duration times (22) of individual pulses and inter-pulse times (23) between pulses;
wherein the computed tomography system (1) is configured to acquire scan data from the subject (5) or part of the subject (5) during the inter-pulse times (23).

2. The radiation therapy system according to claim 1,
wherein the computed tomography system (1) is configured to reconstruct image data, in particular 3D image data, of the subject (5) or part of the subject (5) based on the scan data acquired during inter-pulse times (23).

3. The radiation therapy system according to any one of the preceding claims,
wherein the computed therapy system is configured to activate the x-ray sources (15) only during inter-pulse times (23) and pause acquisition during pulse duration times (22).

4. The radiation therapy system according to claim 1 or 2,
wherein the radiation therapy system is configured to discard scan data acquired during pulse duration times (22) .

5. The radiation therapy system according to any one of the preceding claims,
wherein the radiation therapy system is configured to interleave individual treatment pulses with groups of computed tomography projections (11) such that each group comprises at least one computed tomography projection (11), preferably a plurality of computed tomography projections (11),
wherein a computed tomography projection (11) comprises an activation of at least one of the x-ray sources (15) to create at least one x-ray beam and a detection of the least one x-ray beam projected through the treatment area (4) by at least one of the detector elements.

6. The radiation therapy system according to any one of the preceding claims,
wherein the x-ray sources (15) comprise field emission nanotube-based cathodes.

7. The radiation therapy system according to any one of the preceding claims,
wherein the radiation therapy device (2) comprises a medical linear accelerator.

8. The radiation therapy system according to any one of the preceding claims,
wherein the computed tomography system (1) is configured to acquire scan data by consecutively activating the x-ray sources (15) in the order that the x-ray sources (15) are arranged around the treatment area (4).

9. The radiation therapy system according to any one of the preceding claims,
wherein the computed tomography system (1) is configured to acquire scan data by activating the x-ray sources (15) in such an order that each two consecutively activated x-ray sources (15) are spaced apart by more than 90° on a circle around the treatment area (4).

10. A method for monitoring a subject (5) or part of a subject (5) during a radiation therapy, the radiation therapy involving pulsed treatment beams (21) aiming at a treatment area (4) with a pulsed beam pattern comprising pulse duration times (22) of individual pulses and inter-pulse times (23) between pulses,
the method comprising the following steps:
(a) acquiring scan data with a computed tomography system (1), the computed tomography system (1) comprising x-ray sources (15) and detector elements distributed around the treatment area (4), from the subject (5) or part of the subject (5) during the inter-pulse times (23) ;
(b) reconstructing image data, in particular 3D image data, of the subject (5) or part of the subject (5) based on the scan data acquired during inter-pulse times (23) .

11. The Method according to claim 10,
wherein a computed tomography system (1) according to any one of claims 1 to 9 is applied.

12. The method according to claim 10 or 11, the method further comprising:
- in step (a), performing a first computed tomography scan by acquiring scan data from the subject (5) or part of the subject (5) during the inter-pulse times (23);
- in step (b), reconstructing image data based on the scan data from the first scan;
- (c) after the first computed tomography scan, performing at least one second computed tomography scan, wherein the at least one second computed tomography scan comprises a lower number of computed tomography projections (11) than the first computed tomography scan; and
- (d) updating the image data based on the scan data from the at least one second computed tomography scan.

13. The method according to claim 12,
wherein the computed tomography projections (11) of the at least one second computed tomography scan are directed at a region of interest (41) that is a sub-region of the region covered by the computed tomography projections (11) of the first computed tomography scan.

14. The method according to claim 10 or 11, the method further comprising:
- in step (a), performing an initial computed tomography scan by acquiring scan data from the subject (5) or part of the subject (5) during the inter-pulse times (23) with a low number of computed tomography projections (11) ;
- in step (b), reconstructing rough image data based on the scan data from the first scan in order to determine an estimate of a position of a region of interest (41);
- (c) after the initial computed tomography scan, performing at least one further computed tomography scan, wherein the at least one further computed tomography scan comprises further computed tomography projections (11) that are optimized to image the region of interest (41) ;
- (d) reconstructing image data based on scan data from the at least one further computed tomography scan.

15. A computer program comprising instructions which, when the program is executed by a control unit of a computed tomography system (1), cause the computed tomography system (1) to carry out the method of any one of claims 10 to 14.
